(19) Europäisches Patentamt
European Patent Office
Office européen des brevets

(11) Veröffentlichungsnummer: **0 489 313 B1**

(12) **EUROPÄISCHE PATENTSCHRIFT**

(45) Veröffentlichungstag der Patentschrift: **09.08.95**

(51) Int. Cl.[6]: **C08K 5/37**, C08L 21/00, C07C 323/12

(21) Anmeldenummer: **91119900.8**

(22) Anmeldetag: **22.11.91**

(54) **Kautschukvulkanisate mit guten mechanischen Eigenschaften und verbessertem Hystereseverhalten.**

(30) Priorität: **04.12.90 DE 4038589**

(43) Veröffentlichungstag der Anmeldung:
**10.06.92 Patentblatt 92/24**

(45) Bekanntmachung des Hinweises auf die Patenterteilung:
**09.08.95 Patentblatt 95/32**

(84) Benannte Vertragsstaaten:
**DE ES FR GB IT**

(56) Entgegenhaltungen:
**EP-A- 0 234 377**
**DE-C- 918 927**
**US-A- 3 662 004**

(73) Patentinhaber: **BAYER AG**
**D-51368 Leverkusen (DE)**

(72) Erfinder: **Scholl, Thomas, Dr.**
**Alte Wipperfürther Strasse 24a**
**D-5060 Bergisch Gladbach 2 (DE)**
Erfinder: **Weidenhaupt, Hermann-Josef, Dr.**
**Pfarrer-Forst-Strasse 3**
**W-5164 Nörvenich (DE)**
Erfinder: **Engels, Hans-Wilhelm, Dr.**
**Dickeicherfeld 12**
**W-5014 Kerpen 3 (DE)**

**EP 0 489 313 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind Kautschukvulkanisate mit guten mechanischen Eigenschaften und verbessertem Hystereseverhalten, die unter Verwendung bestimmter sulfidischer Verstärkungsadditive erhalten werden, sowie die Herstellung solcher Kautschukvulkanisate.

Vulkanisate mit verbessertem Hystereseverhalten sind zwar bekannt, sie besitzen jedoch einige unerwünschte Eigenschaften. So werden in der EP 253 365 Hysterese-Verbesserer auf Basis bestimmter Nitroamine beschrieben. Wegen der Gefahr der Umnitrosierung, besteht jedoch der Wunsch nach Kautschukhilfsmitteln, die frei von Nitro- und Nitrosogruppen sind. Ähnliche Bedenken bestehen auch bei den Nitrosoanilinen der US-PS 4 690 965. Aus der EP 0 366 952 sind weiterhin Kautschukvulkanisate bekannt mit verringerten Hystereseverlusten, die bestimmte Diphenylsulfide enthalten. Nachteilig ist, daß diese Additive in Naturkautschuk unwirksam sind und ihn darüber hinaus abbauen (siehe hierzu US-PS 2 470 948).

Weiterhin werden in der DE-OS 2 255 577 bestimmte Organosilane als Verstärkungsadditive beschrieben, die u.a. zur Verbesserung des Hystereseverhaltens bei Kieselsäure gefüllten Kautschukvulkanisaten beitragen. Nachteilig ist jedoch, daß die Hysterese nicht nur bei erhöhter Temperatur (60-100 °C), sondern auch bei tiefen Temperaturen vermindert wird. Es ist jedoch bekannt, daß geringe Hysterese bei tiefen Temperaturen (0-20 °C) mit einem schlechten Naßrutschverhalten bei Kfz-Reifen korreliert.

Gegenstand der Erfindung sind daher Kautschukvulkanisate hergestellt aus mindestens einem Kautschuk, einem Vernetzer, einem Füllstoff, gegebenenfalls weiteren Kautschukhilfsprodukten, sowie mindestens einem sulfidischen Verstärkungsadditiv der Formel

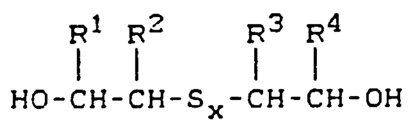

(I) ,

worin

- $R^1$ bis $R^4$ gleich oder verschieden sind und Wasserstoff, $C_1$-$C_6$-Alkyl, $C_5$-$C_6$-Cycloalkyl, $C_6$-$C_{10}$-Aryl oder eine Gruppe $-CH_2-O-R^5$, $-CH_2-CH_2-OR^5$ mit $R^5$ = Wasserstoff, $C_1$-$C_6$-Alkyl, $C_5$-$C_6$-Cylcoalkyl, $C_6$-$C_{10}$-Aryl oder $C_1$-$C_6$-Acyl bedeuten und
- X für eine ganze Zahl von 2 bis 6 steht,

in Mengen von 0,1 bis 15 Gew.-%, bezogen auf Kautschuk.

Als Alkyl-, Cycloalkyl-, Arylreste werden bevorzugt genannt: der Methyl-, Ethyl-, Propyl-, Butyl-, Cyclohexyl- sowie Phenylrest; als $-CH_2-O-R^5$-Gruppe: $-CH_2-O-H$, $-CH_2-O-CH_3$, $-CH_2-O-C_6H_5$.

Zur Herstellung der Verbindungen (I) geht man beispielsweise von Mercaptoethanol, Mercaptopropanol oder Thioglycerin aus, die mit Schwefeldichlorid bzw. Dischwefelchlorid zu Verbindungen (I) mit x = 3 bzw. x = 4 umgesetzt werden können (siehe Reaktionsschema A):

<u>Reaktion A:</u>

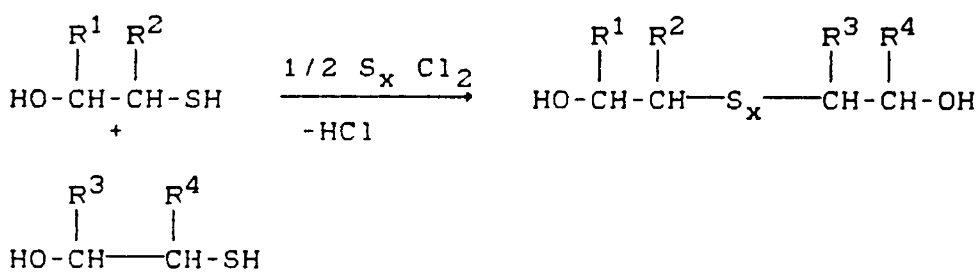


(I)

Zur Durchführung der Reaktionen siehe z.B. Houben-Weyl, Methoden der organischen Chemie, Band 9, Seite 88 ff (1955) und Band E11 (1985), Thieme Verlag, Stuttgart.

Disulfide gemäß Formel (I) (mit x = 2) erhält man beispielsweise durch Oxidation der Mercaptane gemäß Reaktionsschema B:

Reaktion B:

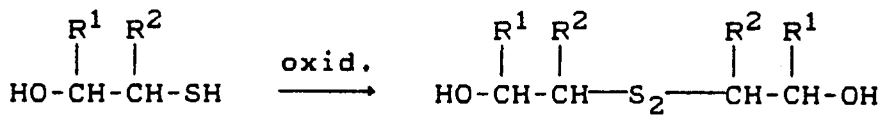


(I)

Geeignete Oxidationsmittel sind beispielsweise Wasserstoffperoxid, Sauerstoff, Dimethylsulfoxid, Stickstoffmonoxid, Chlor, Jod (siehe hierzu Houben-Weyl, Methoden der organischen Chemie, Band IX, Seite 60 ff., 1955, Thieme Verlag, Stuttgart).

Eine weitere Methode zur Variation der Reste $R^1$-$R^4$ beruht auf der Ringöffnung von Alkyl- oder Arylsubstituierten Glycidylethern mit Schwefelwaserstoff oder Natriumsulfid zu Verbindungen des Typs II mit $R^1$, $R^2$ = Alkyl, Aryl oder $CH_2$-O-$R^5$ (siehe Reaktionsschema C):

Reaktion C:

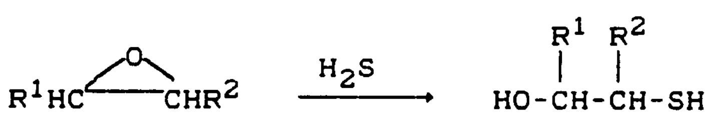


Anschließende Umsetzung mit Schwefeldichlorid bzw. Dischwefeldichlorid führt zu entsprechenden Verbindungen des Typs I (siehe Reaktionsschema D)

Reaktion D:

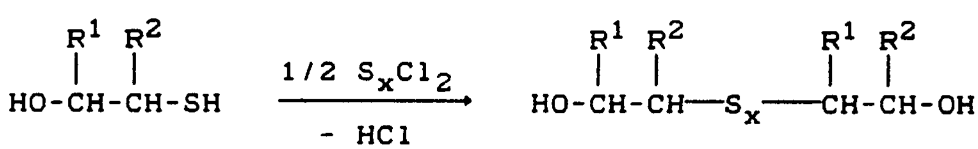


(I)

In besonders vorteilhafter Weise gelingt die Herstellung der Verbindungen der Formel (I), wenn man Hydroxygruppen-haltige Alkylhalogenide mit Alkalipolysulfiden in Gegenwart von alkoholischen Lösungsmitteln bei Temperaturen von -20 °C bis 120 °C, bevorzugt bei 20 bis 80 °C, umsetzt (s. Reaktionsschema E am Beispiel der Umsetzung eines Hydroxygruppen-haltigen Alkylhalogenids mit Natriumpolysulfid (hergestellt aus $Na_2S$ und Schwefel).

Reaktion E:

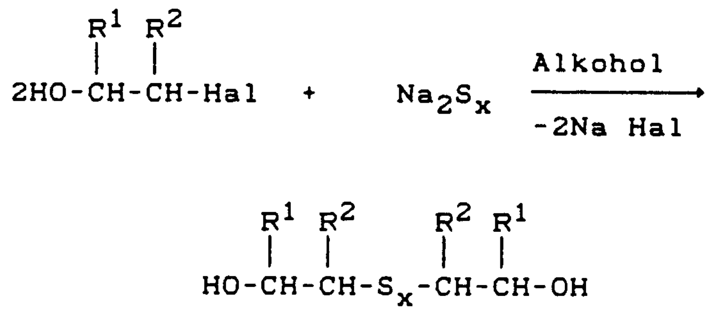


(I)

Als Alkalipolysulfide können solche der Formel $Me_2S_x$ eingesetzt werden, in der Me für Lithium, Natrium oder Kalium steht und x eine ganze Zahl von 2 bis 6 bedeutet.

Als Alkohole können eingesetzt werden:

- Methanol, Ethanol, n-Propanol, i-Propanol, n-Butanol, i-Butanol Amylalkohol, Hexylalkohol, n-Octanol, Ethylhexylalkohol,
- Ethylenglykol, 1,2- und 1,3-Propylenglykol, 1,4-Butandiol, 1,6-Hexandiol,
- Glycerin, Trimethylolpropan, Pentaerythrit,
- Diethylenglykol, Triethylenglykol, Tetraethylenglykol, Octaethylenglykol, Di-, Tri- und Tetrapropylenglykol,
- Ethylenglykolmonomethylether, Diethylenglykolmonomethylether, Triethylenglykolmonomethylether, Propylenglykolmonomethylether, Dipropylenglykolmonomethylether

sowie deren Gemische.

Die Menge an Alkohol wird so bemessen, daß sich das Alkalipolysulfid im Alkohol löst. Ein Alkoholüberschuß ist nicht kritisch.

Als Hydroxygruppen-haltige Alkylhalogenide können eingesetzt werden:

- 1-Hydroxy-2-chlorethan, 2-Hydroxy-1-chlorpropan, 1-Hydroxy-2-chlorpropan, 1,2-Dihydroxy-3-chlorpropan, 1,3-Dihydroxy-2-chlorpropan, 1,4-Dihydroxy-2-chlorbutan, 1,2,4-Trihydroxy-3-chlorpropan sowie die entsprechenden Fluoride, Bromide oder Jodide. Besonders bevorzugt werden 1,2-Dihydroxy-3-chlorpropan und 1,3-Dihydroxy-2-chlorpropan sowie 1,4-Dihydroxy-2-chlorbutan eingesetzt.

Die nach diesem Verfahren erhaltenen Bis(hydroxyalkyl)-polysulfide (I) haben einen Schwefelgehalt (nach Elementaranalyse) von ca. 32-46%, entsprechend einem $-S_x-$ mit x = 2,4-4,0.

Die Umsetzung wird so vorgenommen, daß man zuerst in bekannter Weise aus einem Alkalisulfid und Schwefel bei Temperaturen von ca, 0-120 °C ein Alkalipolysulfid herstellt und dieses anschließend bei Temperaturen von -20 °C bis +120 °C mit Hydroxygruppen-haltigen Alkylhalogeniden zur Reaktion bringt. Die Reaktion ist in den meisten Fällen leicht exotherm. Die Reaktionszeiten liegen im Bereich von ca. 1 bis 10 Stunden. Das abgeschiedene Alkalihalogenid kann danach abfiltriert werden.

Bei den Verbindungen der Formel (I) handelt es sich in der Regel um Flüssigkeiten, die erst nach längerem Stehen bei Raumtemperatur auskristallisieren.

Als Beispiele für Verbindungen der Formel (I) seien folgende Verbindungen aufgeführt:

$HO-CH_2CH_2-S_3-CH_2CH_2OH$, $HOCH_2CH_2-S_4-CH_2CH_2OH$,

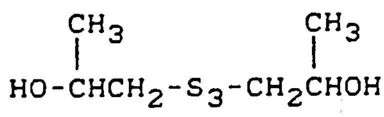 ,

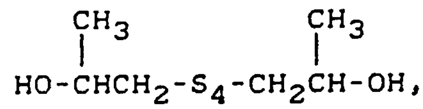

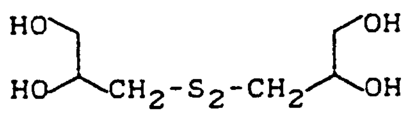 ,

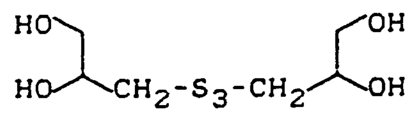 ,

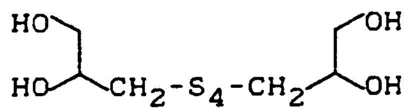 ,

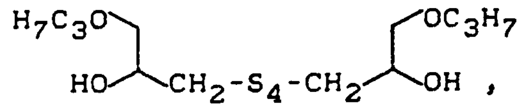 ,

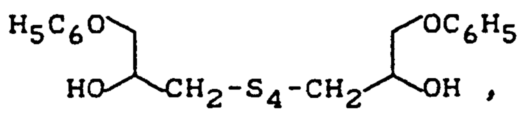 ,

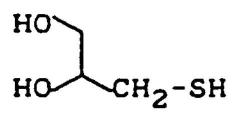

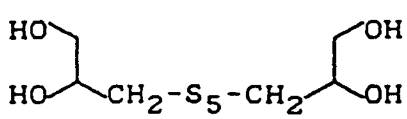 ,

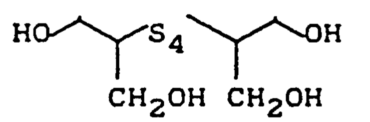 .

Besonders bevorzugt sind:

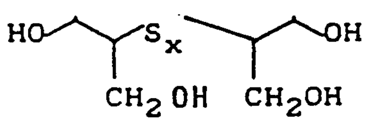 ,

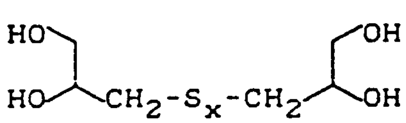 ,

x = 2 - 5

$HO-CH_2CH_2-S_x-CH_2CH_2-OH$

x = 3 - 5 .

Die sulfidischen Verbindungen der Formel (I) werden bevorzugt in Mengen von 0,1 bis 7,5 % bezogen auf Kautschuk, bei der Herstellung der Kautschukvulkanisate eingesetzt.

Ein weiterer Gegenstand der vorliegenden Erfindung ist ein Verfahren zur Herstellung von gefüllten Kautschukvulkanisaten, das dadurch gekennzeichnet ist, daß man

i) mindestens einen Kautschuk mit

ii) 10 - 120, vorzugsweise 30 - 80 Gew.-%, bezogen auf Kautschuk (i), Füllstoff und

iii) 0,1 - 15, vorzugsweise 0,1 - 7,5 Gew.-%, bezogen auf Kautschuk (i), sulfidischer Verbindungen der Formel

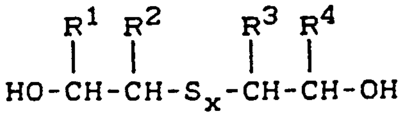 (I) ,

worin

$R^1$ bis $R^4$ gleich oder verschieden sind und Wasserstoff, $C_1$-$C_6$-Alkyl, $C_5$-$C_6$-Cycloalkyl, $C_6$-$C_{10}$-Aryl oder eine Gruppe $-CH_2-O-R^5$, $-CH_2-CH_2-OR^5$ mit $R^5$ = Wasserstoff, $C_1$-$C_6$-Alkyl, $C_5$-$C_6$-Cylcoalkyl, $C_6$-$C_{10}$-Aryl oder $C_1$-$C_6$-Acyl bedeuten und

X für eine ganze Zahl von 2 bis 6 steht,

bei Massetemperaturen von mindestens 120 °C und bei Scherraten von 1 - 1000 $sec^{-1}$, bevorzugt 1 - 100 $sec^{-1}$, mischt und anschließend nach Zugabe weiterer Vulkanisationschemikalien in üblicher Weise vulkanisiert.

Die Zugabe der erfindungsgemäßen Additive der Formel (I) sowie die Zugabe der Füllstoffe erfolgt bevorzugt im ersten Teil des Mischprozesses bei Massetemperaturen von 100 - 200 °C bei den erwähnten Scherraten, sie kann jedoch auch später bei tieferen Temperaturen (40-100 °C) z.B. zusammen mit Schwefel und Beschleuniger erfolgen.

Die erfindungsgemäßen Additive der Formel (I) können sowohl in reiner Form als auch aufgezogen auf einen inerten, organischen oder anorganischen Träger dem Mischprozeß zugegeben werden. Bevorzugte Trägermaterialien sind Kieselsäuren, natürliche oder synthestische Silikate, Aluminiumoxid und/oder Russe.

Als Füllstoffe kommen für die erfindungsgemäßen Kautschukvulkanisate in Frage:

- hochdisperse Kieselsäuren, hergestellt z.B. durch Fällung von Lösungen von Silikaten oder Flammenhydrolyse von Siliciumhalogeniden mit spezifischen Oberflächen von 5-1000, vorzugsweise 20-400 $m^2/g$ (BET-Oberfläche) und mit Primärteilchengrößen von 100-400 nm. Die Kieselsäuren können gegebenenfalls auch als Mischoxide mit anderen Metalloxiden wie Al-, Mg-, Ca-, Ba-, Zn-, Zr-, Ti-Oxiden vorliegen.
- Synthetische Silikate, wie Aluminiumsilikat, Erdalkalisilikate wie Magnesium- oder Calciumsilikat, mit BET-Oberflächen von 20-400 $m^2/g$ und Primärteilchendurchmesser von 10-400 nm.
- Natürliche Silikate, wie Kaolin und andere natürlich vorkommende Kieselsäuren.
- Glasfasern und Glasfaserprodukte (Matten, Stränge) oder Mikroglaskugeln.

Als weitere Füllstoffe können Russe verwendet werden. Die hierbei zu verwendenden Russe sind z.B. nach dem Flammruß, Furnace- oder Gasruß-Verfahren hergestelltund besitzen BET-Oberflächen von 20-200 $m^2/g$, wie SAF-, ISAF-, IISAF-, HAF-, FEF- oder GPF-Russe.

Eine besonders bevorzugte Variante besteht in der Kombination von Kieselsäuren, Ruß und Verstärkungsadditive der Formel (I). Bei dieser Kombination kann das Verhältnis von Kieselsäure zu Ruß in beliebigen Grenzen variiert werden. Aus reifentechnischer Sicht werden z.B. Kieselsäure: Ruß-Verhältnisse von 1:10 bis 1:2 bevorzugt.

Für die Herstellung erfindungsgemäßer Kautschukvulkanisate eigenen sich neben Naturkautschuk auch Synthesekautschuke. Bevorzugte Synthesekautschuke sind beispielsweise bei W. Hofmann, Kautschuk-Technologie, Gentner-Verlag, Stuttgart 1980, beschrieben, Sie umfassen u.a.

BR - Polybutadien
ABR - Butadien/Acrylsäure-$C_{1-4}$-alkylester-Copolymerisate
CR - Polychloropren
IR - Polyisopren
SBR - Styrol/Butadien-Copolymerisate mit Styrolgehalten von 1- 60, vorzugsweise 20-50 Gew.-%
IIR - Isobutylen/Isopren-Copolymerisate
NBR - Butadien/Acrylnitril-Copoylmerisate mit Acrylnitrilgehalten von 5-60, vorzugsweise 10-50 Gew.-%
HNBR - teilhydrierter oder vollständig hydrierter NBR-Kautschuk
EPDM - Ethylen/Propylen/Dien-Copolymerisate

sowie Mischungen dieser Kautschuke.

Die erfindungsgemäßen Kautschukvulkanisate können weitere Kautschukhilfsprodukte enthalten, wie Reaktionsbeschleuniger, Alterungsschutzmittel, Wärmestabilisatoren, Lichtschutzmittel, Ozonschutzmittel, Verarbeitungshilfsmittel, Weichmacher, Tackifier, Treibmittel, Farbstoffe, Pigmente, Wachse, Streckmittel, organische Säuren, Verzögerer, Metalloxide sowie Aktivatoren, wie Triethanolamin, Polyethylenglykol, Hexantriol, die der Gummiindustrie bekannt sind.

Die Kautschukhilfsprodukte werden in üblichen Mengen, die sich u.a. nach dem Verwendungszweck der Vulkanisate richten, eingesetzt. Übliche Mengen sind 0,1 bis 30 Gew.-%, bezogen auf Kautschuk.

Als Vernetzungsmittel werden eingesetzt Peroxide, Schwefel, Magnesiumoxid, Zinkoxid, denen noch die bekannten Vulkanisationsbeschleuniger, wie Mercaptobenzthiazole, -sulfenamide, Thiurame und Thiocarbonate zugesetzt werden können. Bevorzugt ist Schwefel.

Die Vernetzungsmittel und Vulkanisationsbeschleuniger werden in Mengen von ca. 0,1 bis 10 Gew.-%, bevorzugt 0,1 bis 5 Gew.-%, bezogen auf Kautschuk, eingesetzt.

Die Vulkanisation kann bei Temperaturen von 100-200 °C, vorzugsweise 130-180 °C, gegebenenfalls unter einem Druck von 10-200 bar, erfolgen.

Die Abmischung des Kautschuks mit dem Füllstoff und den sulfidischen Verstärkungsaddtiven der Formeln (I) und/oder (II) kann in üblichen Mischaggregaten, wie Walzen, Innenmischer und Mischextruder, durchgeführt werden.

Gegenstand der Erfindung sind weiterhin neue Oligosulfide der Formeln:

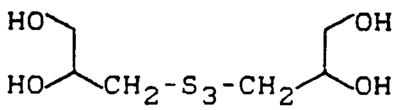 ,

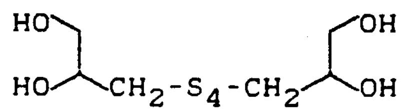 ,

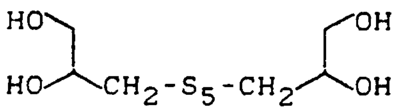

und

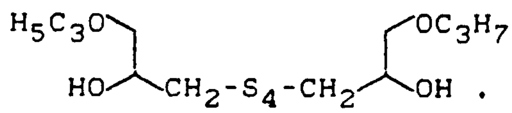

Die erfindungsgemäßen Kautschukvulkanisate eignen sich zur Herstellung von Formkörpern, z.B. für die Herstellung von Kabelmänteln, Schläuchen, Treibriemen, Förderbändern, Walzenbelägen, Reifen, Schuhsohlen, Dichtungsringen und Dämpfungselementen.

## Beispiele

### Beispiel 1:

Herstellung von:

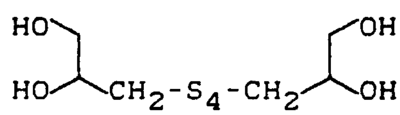

108 g (1,0 Mol) Thioglycerin werden in 150 ml Isopropanol gelöst und bei 0-5 °C mit 67,5 g (0,5 Mol) Dischwefeldichlorid versetzt. Unter Durchleiten eines Stickstoffstroms rührt man 12 Stunden bei Raumtemperatur nach und entfernt dann das Lösungsmittel bei 50 °C im Vakuum. Man erhält ein gelbliches hochviskoses Öl, das nach längerem Stehen bei Raumtemperatur auskristallisiert. Fp.: 43-45 °C.

| | | |
|---|---|---|
| Elementaranalyse: | C: | 25,1 (ber. 25,9) |
| | H: | 5,1 (ber. 5,0) |
| | S: | 45,1 (ber. 46,0) |

Das Produkt enthält nach IR keine freien Mercaptogruppen.

Beispiel 2:

Herstellung von:

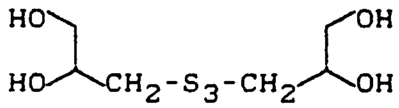

Verfährt man wie in Beispiel 1 unter Verwendung von 108 g (1,0 Mol) Thioglycerin und 51 g (0,5 Mol) Schwefeldichlorid, so erhält man 123 g eines gelben Öls. Nach Kristallisation: Fp. 34-37 ° C.

| | | |
|---|---|---|
| Elementaranalyse: | C:<br>H:<br>S: | 29,3 (ber. 29,3)<br>5,6 (ber. 5,7)<br>39,1 (ber. 39,0) |

Beispiel 3:

Herstellung von:

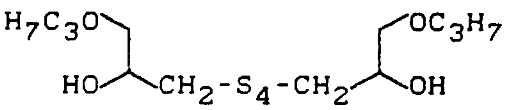

150 g (1,0 Mol) 2-Hydroxy-3-mercapto-dipropylether, hergestellt aus Glycidylisopropylether und Natriumsulfid, Kp. 130 ° C/0,1 mm, werden gemäß dem Verfahren aus Beispiel 1 mit 67,5 g (0,5 Mol) Dischwefeldichlorid umgesetzt. Man erhält 182 g eines gelben Öls. Das Produkt ist laut IR-Spektroskopie frei von Mercaptangruppen.

| | | |
|---|---|---|
| Elementaranalyse: | C:<br>H:<br>S: | 39,4 (ber. 39,8)<br>7,3 (ber. 7,2)<br>35,7 (ber. 35,4) |

Beispiel 4:

Herstellung von: $HO-CH_2-CH_2-S_4-CH_2-CH_2-OH$

Analog dem Verfahren gemäß Beispiel 1 aus 1,0 Mol Mercaptoethanol und 0,5 Mol Dischwefeldichlorid.

Gelbes viskoses Öl.

| | | |
|---|---|---|
| Elementaranalyse: | C:<br>H:<br>S: | 22,2 (ber. 22,02)<br>4,7 (ber. 4,59)<br>57,9 (ber. 58,72) |

Beispiel 5:

Herstellung von Bis-(1,2-dihydroxypropyl)-oligosulfid in Isopropanol

320 g (10 mol) Schwefel werden in 2 l Isopropanol dispergiert. Dann setzt man bei 60 °C 264.4 g (2 mol) Natriumsulfid-trihydrat hinzu und rührt ca. eine Stunde bei dieser Temperatur bis sich eine klare Lösung von Natriumpolysulfid gebildet hat. Anschließend tropft man innerhalb von 1,5 Stunden 440 g (4 mol) 1,2-Dihydroxy-3-chlorpropan hinzu und rührt ca. 6 h bei 60 °C nach. Nach dem Abkühlen auf Raumtemperatur wird das abgeschiedene Natriumchlorid zusammen mit überschüssigem Schwefel abfiltriert. Nach dem Eindampfen werden 515 g eines hochviskosen gelben Öls erhalten.
Schwefelgehalt nach Elementaranalyse: 41,2%.

Das Produkt besteht nach LC-MS-Analyse aus einem Gemischt von:
5,6% Disulfid
41,4% Trisulfid
31,2% Tetrasulfid
12,9% Pentasulfid
6,6% Hexasulfid
2,3% Heptasulfid

Beispiele 6-7:

Verfährt man wie in Beispiel 6 und variiert hierbei das Lösungsmittel, so erhält man folgende Produkte:

| Beispiel Nr. | Lösungsmittel | Ausbeute | S-Gehalt |
|---|---|---|---|
| 6 | Ethanol | 507 g | 39.0% |
| 7 | n-Butanol | 526 g | 41.6% |
| Vergleichsbeispiel FR-PS 2099583 | Wasser | 424 g | 32.9% |

Beispiele 8-10: (Herstellung von Kautschukvulkanisaten)

In einem Kneter werden bei 140 °C Innentemperatur und einer Drehzahl von 50 Upm Kautschuk-Mischungen der nachfolgenden Zusammensetzung hergestellt. Die Mischzeit betrug 5 Minuten. Die Zugabe von Schwefel und Beschleuniger erfolgte bei 50 °C auf einer Walze. Die Vulkanisation wurde bei 150 °C innerhalb von 20 Minuten durchgeführt.

Kautschukmischung:

| | |
|---|---|
| Naturkautschuk TSR 5 | 100 Gew.-Teile |
| der Fa. Malaysian Rubber Stearinsäure | 2 Gew.-Teile |
| Zinkoxid | 3 Gew.-Teile |
| oligomeres Trimethyldihydrochinolin (Vulkanox HS, Fa. Bayer) | 1,5 Gew.-Teile |
| N-Isopropyl-N'-phenyl-p-phenylendiamin | 1,5 Gew.-Teile |
| (Vulkanox 4040 NA, Fa. Bayer) Morpholin-mercaprobenzthiazol (Vulkazit MOZ, Fa. Bayer) | 1,5 Gew.-Teile |
| Schwefel | 1,2 Gew.-Teile |

Dieser Mischung wurden als Füllstoffe Ruß und Kieselsäure sowie verschiedene sulfidische Verstärkungsadditive zugesetzt. Die Mengenverhältnisse sind der nachfolgenden Tabelle zu entnehmen. Bezeichnung Ruß bezieht sich auf Ruß des Typs N 110, und die Bezeichnung Kieselsäure ($SiO_2$) bezieht sich auf Vulkasil S der Fa. Bayer. Die Mengenverhältnisse sind in Gew.-Teile angegeben.

Tabelle 1

| | Bsp. 8 | Bsp. 9 | Bsp. 10 | Vergl. 1 | Vergl. 2 | Vergl. 3 |
|---|---|---|---|---|---|---|
| | 30 Ruß<br>18 $SiO_2$<br>3 Bsp. 1* | 30 Ruß<br>18 $SiO_2$<br>3 Bsp. 2 | 30 Ruß<br>18 $SiO_2$<br>3 Bsp. 4 | 30 Ruß<br>18 $SiO_2$<br>3 TESPT** | 30 Ruß<br>18 $SiO_2$ | 48 Ruß |
| Zugfestigkeit [MPa] | 27 | 26 | 26 | 26 | 22 | 27 |
| Bruchdehnung [%] | 582 | 621 | 623 | 562 | 625 | 545 |
| Weiterreiß-festigkeit [N/mm] | 489 | 569 | 454 | 468 | 435 | 487 |
| Modul 100 [MPa] | 2.3 | 2.2 | 2.0 | 2.2 | 1.4 | 2.6 |
| Härte [Shore A] | 66 | 61 | 66 | 65 | 58 | 69 |
| Elastizität [%] $20^0/70^0$ C | 51/63 | 50/62 | 46/60 | 50/61 | 47/56 | 44/57 |
| Tan δ (DIN 53535) | | | | | | |
| $20^0$ C | 0.127 | 0.126 | 0.140 | 0.115 | 0.130 | 0.140 |
| $60^0$ C | 0.079 | 0.082 | 0.094 | 0.081 | 0.095 | 0.110 |

* Verbindung des aufgeführten Beispiels
** Bis-(triethoxysilylpropyl)-tetrasulfid gemäß DE-OS 2 255 577

Aus den dargestellten Ergebnissen der Tabelle wird deutlich, daß Kautschukvulkanisate, die die erfindungsgemäßen Verstärkeradditive enthalten, ein überlegenes Temperatur/Wirkungsverhalten aufweisen, d.h. sie beeinflußen den tan δ bei Raumtemperatur nur geringfügig, während sie ihn bei höherer Temperatur erheblich senken. Gegenüber der Additiv-freien Mischung weisen sie noch erhebliche Vorteile im mechanischen Verhalten auf.

Beispiel 11:

Rußgefüllte Kautschukvulkanisate:

| | |
|---|---|
| Naturkautschuk TSR 5 der Fa. Malaysian Rubber | 100 Gew.-Teile |
| Ruß N110 der Fa. Degussa | 48 Gew.-Teile |
| Stearinsäure | 2,0 Gew.-Teile |
| Paraffinwachs | 1,0 Gew.-Teile |
| oligomeres Trimethyldihydrochinolin | 1,0 Gew.-Teile |
| N-Isopropyl-N'-phenyl-p-phenylendiamin | 1,4 Gew.-Teile |
| Additiv gem. Beispiel 1 | 1,5 Gew.-Teile |
| Zinkoxid | 3,5 Gew.-Teile |
| Schwefel | 1,2 Gew.-Teile |
| N-tert.-Butyl-mercaptobenzthiazolsulfenamid | 1,4 Gew.-Teile |

Diese Mischung wird im Kneter bei 160 °C Innentemperatur (Drehzahl 50 Upm) hergestellt. Schwefel und Beschleuniger werden auf der Walze bei 50 °C nachgemischt. Die Vulkanisationsbedingungen sind 150 °C/20 Minuten.

| | Beispiel 11 (mit 1,5 Teilen der Verbindung des Beispiels 1) | Vergleich ohne Additiv |
|---|---|---|
| Modul 300 [MPa] | 15,3 | 12,5 |
| Festigkeit [MPa] | 25,2 | 25,9 |
| Dehnbarkeit [%] | 475 | 545 |
| Härte [Shore A] | 68 | 66 |
| tan δ | | |
| 20 °C | 0,198 | 0,190 |
| 60 °C | 0,095 | 0,106 |

Auch an dieser ausschließlich mit Ruß gefüllten Mischung wird das günstige Temperatur/Wirkungsverhalten der erfindungsgemäßen Kautschukadditive deutlich: Verschlechterung des Hystereseverhaltens bei tiefer Temperatur und Verbesserung bei erhöhter Temperatur.

Beispiel 12

Kieselsäure-gefüllte Kautschukvulkanisate:

| | |
|---|---|
| SBR-Kautschuk (Buna EM 1500) | 70 Gew.-Teile |
| SBR-Kautschuk (Buna EM 1778) | 41 Gew.-Teile |
| Kieselsäure (Vulkasil S) | 50 Gew.-Teile |
| Zinkoxid | 3 Gew.-Teile |
| Stearinsäure | 2 Gew.-Teile |
| Oktyliertes Diphenylamin | 1 Gew.-Teil |
| Cumaronharz | 5 Gew.-Teile |
| Additiv gemäß Bsp. 1 und 5 | 3,5 Gew.-Teile |
| Morpholin-mercaptobenzthiazolsulfenamid | 1,5 Gew.-Teile |
| Tetramethylthiuramdisulfid | 0,1 Gew.-Teile |
| Schwefel | 2 Gew.-Teile |

Diese Mischung wird im Kneter bei 150 °C Innentemperatur (Drehzahl ca. 50 Kpm) hergestellt. Schwefel und Beschleuniger werden auf der Walze bei 50 °C nachgemischt. Die Additiv-freie Vergleichsmischung enthielt 0,3 Gew.-Teile Tetramethylthiuramdisulfid um einen vergleichbaren Vernetzungsgrad einzustellen.

Die Vulkanisationsbedingungen sind 160 °C/15 Minuten.

| | Beispiel 12 | Vergleich 1 | Vergleich 2 |
|---|---|---|---|
| | mit Verb. gem. Bsp. 1 | ohne Additiv | mit TESPT gem. DE-OS 2 255 577 |
| Zugfestigkeit [MPa] | 16 | 15 | 20 |
| Bruchdehnung [%] | 577 | 819 | 543 |
| Modul 100 [MPa] | 2,2 | 1,1 | 2,2 |
| Härte [Shore A] | 66 | 57 | 66 |
| Weiterreißfestigkeit [N/mm] | 178 | 216 | 190 |
| Elastizität [%] | 45 | 41 | 44 |

Ergebnis: Mit den erfindungsgemäßen Substanzen läßt sich die Härte, Elastizität und der Modul gegenüber dem additivfreien Vulkanisat erheblich steigern. Gegenüber der TESPT-haltigen Probe werden darüber hinaus Vorteile in der Elastizität gefunden.

**Patentansprüche**

**Patentansprüche für folgende Vertragsstaaten : DE, FR, GB, IT**

1. Kautschukvulkanisate hergestellt aus mindestens einem Kautschuk, einem Vernetzer, einem Füllstoff, gegebenenfalls weiteren Kautschukhilfsprodukten sowie mindestens einem sulfidischen Verstärkungsadditiv der Formel

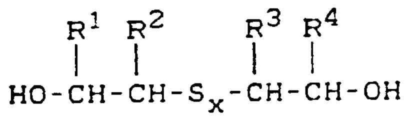

,

worin

$R^1$ bis $R^4$ gleich oder verschieden sind und Wasserstoff, $C_1$-$C_6$-Alkyl, $C_5$-$C_6$-Cycloalkyl, $C_6$-$C_{10}$-Aryl oder eine Gruppe $-CH_2-O-R^5$, $-CH_2-CH_2-OR^5$ mit $R^5$ = Wasserstoff, $C_1$-$C_6$-Alkyl, $C_5$-$C_6$-Cylcoalkyl, $C_6$-$C_{10}$-Aryl oder $C_1$-$C_6$-Acyl bedeuten und

X für eine ganze Zahl von 2 bis 6 steht,

in Mengen von 0,1 bis 15 Gew.-%, bezogen auf Kautschuk.

2. Verfahren zur Herstellung von gefüllten Kautschukvulkanisaten, dadurch gekennzeichnet, daß man

i) mindestens einen Kautschuk mit

ii) 10 - 120, vorzugsweise 30 - 80 Gew.-%, bezogen auf Kautschuk (i), Füllstoff und

iii) 0,1 - 15, vorzugsweise 0,1 - 7,5 Gew.-%, bezogen auf Kautschuk (i), sulfidischer Verbindungen der Formel

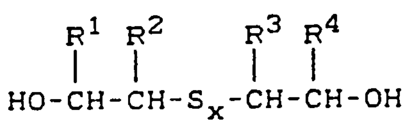

,

worin

$R^1$ bis $R^4$ gleich oder verschieden sind und Wasserstoff, $C_1$-$C_6$-Alkyl, $C_5$-$C_6$-Cycloalkyl, $C_6$-$C_{10}$-Aryl oder eine Gruppe $-CH_2-O-R^5$, $-CH_2-CH_2-O-R^5$ mit $R^5$ = Wasserstoff, $C_1$-$C_6$-Alkyl, $C_5$-$C_6$-Cylcoalkyl, $C_6$-$C_{10}$-Aryl oder $C_1$-$C_6$-Acyl bedeuten und

X für eine ganze Zahl von 2 bis 6 steht;

bei Massetemperaturen von mindestens 120 °C und bei Scherraten von 1 - 1000 $sec^{-1}$ mischt und anschließend in üblicher Weise vulkanisiert.

3. Oligosulfide der Formeln

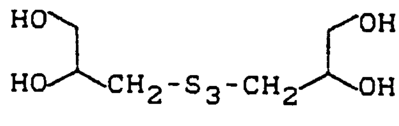 , 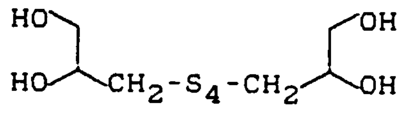 ,

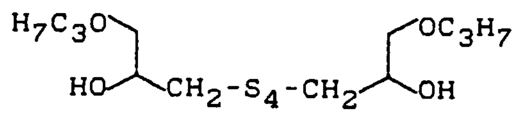 ,

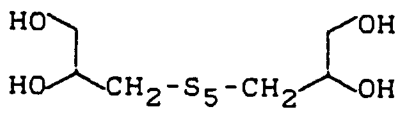 ,

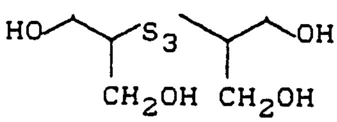 und 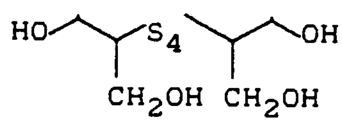 .

**Patentanspruch für folgenden Vertragsstaat : ES**

1. Verfahren zur Herstellung von gefüllten Kautschukvulkanisaten, dadurch gekennzeichnet, daß man
   i) mindestens einen Kautschuk mit
   ii) 10 - 120, vorzugsweise 30 - 80 Gew.-%, bezogen auf Kautschuk (i), Füllstoff und
   iii) 0,1 - 15, vorzugsweise 0,1 - 7,5 Gew.-%, bezogen auf Kautschuk (i), sulfidischer Verbindungen der Formel

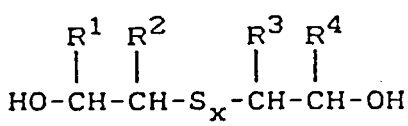 ,

   worin
   $R^1$ bis $R^4$ gleich oder verschieden sind und Wasserstoff, $C_1$-$C_6$-Alkyl, $C_5$-$C_6$-Cycloalkyl, $C_6$-$C_{10}$-Aryl oder eine Gruppe $-CH_2-O-R^5$, $-CH_2-CH_2-O-R^5$ mit $R^5$ = Wasserstoff, $C_1$-$C_6$-Alkyl, $C_5$-$C_6$-Cylcoalkyl, $C_6$-$C_{10}$-Aryl oder $C_1$-$C_6$-Acyl bedeuten und
   X für eine ganze Zahl von 2 bis 6 steht,
   bei Massetemperaturen von mindestens 120 °C und bei Scherraten von 1 - 1000 $sec^{-1}$ mischt und anschließend in üblicher Weise vulkanisiert.

**Claims**
**Claims for the following Contracting States : DE, FR, GB, IT**

1. Rubber vulcanisates prepared from at least one rubber, a cross-linking agent, a filler, optionally other auxiliary substances for rubber and at least one sulphide-type reinforcing additive corresponding to the formula

$$\begin{array}{cccc} R^1 & R^2 & R^3 & R^4 \\ | & | & | & | \\ \text{HO-CH} & \text{- CH-}S_x\text{-CH} & \text{-} & \text{CH-OH} \end{array}$$

wherein

- $R^1$ to $R^4$ are identical or different and signify hydrogen, $C_1$-$C_6$ alkyl, $C_5$-$C_6$ cycloalkyl, $C_6$-$C_{10}$ aryl or a group $-CH_2-O-R^5$, $-CH_2-CH_2-OR^5$ wherein $R^5$ represents hydrogen, $C_1$-$C_6$ alkyl, $C_5$-$C_6$ cycloalkyl, $C_6$-$C_{10}$ aryl or $C_6$-$C_{10}$ acyl and
- X represents an integer from 2 to 6,

in quantities of from 0.1 to 15% by weight, referred to rubber.

**2.** Process for the preparation of filled rubber vulcanisates, characterised in that
i) at least one rubber is mixed with
ii) from 10 to 120% by weight, preferably from 30 to 80% by weight, referred to rubber (i), of filler and
iii) from 0.1 to 15% by weight, preferably from 0.1 to 7.5% by weight, referred to rubber (i), of sulphide-type compounds corresponding to the formula

$$\begin{array}{cccc} R^1 & R^2 & R^3 & R^4 \\ | & | & | & | \\ \text{HO-CH} & \text{- CH-}S_x\text{-CH} & \text{-} & \text{CH-OH} \end{array}$$

wherein

- $R^1$ to $R^4$ are identical or different and signify hydrogen, $C_1$-$C_6$ alkyl, $C_5$-$C_6$ cycloalkyl, $C_6$-$C_{10}$ aryl or a group $-CH_2-O-R^5$, $-CH_2-CH_2-OR^5$ wherein $R^5$ represents hydrogen, $C_1$-$C_6$ alkyl, $C_5$-$C_6$ cycloalkyl, $C_6$-$C_{10}$ aryl or $C_6$-$C_{10}$ acyl and
- X represents an integer from 2 to 6,

at temperatures of the mixture of at least 120 °C and at shear rates of from 1 to 1000 $sec^{-1}$, and the mixture is then vulcanised in the conventional manner.

3. Oligosulphides corresponding to the formulae

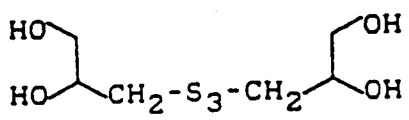 , 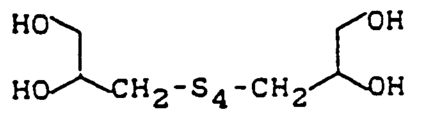 ,

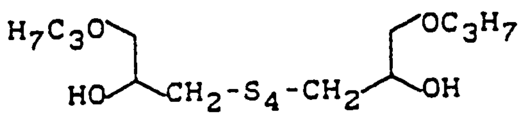 ,

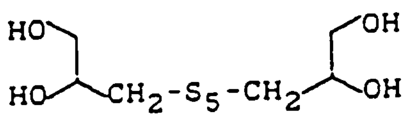 ,

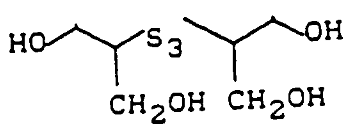 and 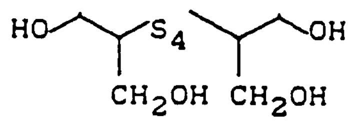 .

**Claim for the following Contracting State : ES**

1. Process for the preparation of filled rubber vulcanisates, characterised in that
   i) at least one rubber is mixed with
   ii) from 10 to 120% by weight, preferably from 30 to 80% by weight, referred to rubber (i), of filler and
   iii) from 0.1 to 15% by weight, preferably from 0.1 to 7.5% by weight, referred to rubber (i), of sulphide-type compounds corresponding to the formula

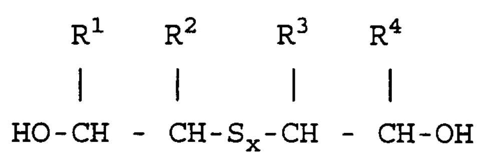

wherein

$R^1$ to $R^4$ are identical or different and signify hydrogen, $C_1$-$C_6$ alkyl, $C_5$-$C_6$ cycloalkyl, $C_6$-$C_{10}$ aryl or a group $-CH_2$-$O$-$R^5$, $-CH_2$-$CH_2$-$OR^5$ wherein $R^5$ represents hydrogen, $C_1$-$C_6$ alkyl, $C_5$-$C_6$ cycloalkyl, $C_6$-$C_{10}$ aryl or $C_6$-$C_{10}$ acyl and

X represents an integer from 2 to 6,

at temperatures of the mixture of at least 120 °C and at shear rates of from 1 to 1000 $sec^{-1}$, and the mixture is then vulcanised in the conventional manner.

**Revendications**

**Revendications pour les Etats contractants suivants : DE, FR, GB, IT**

1. Vulcanisats de caoutchoucs préparés à partir d'au moins un caoutchouc, d'un agent réticulant, d'une matière de charge, le cas échéant d'autres produits auxiliaires pour caoutchoucs et d'au moins un additif renforçant sulfuré de formule:

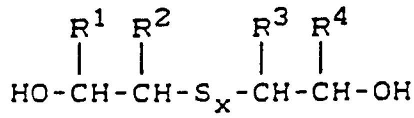

dans laquelle

$R^1$ à $R^4$, ayant des significations identiques ou différentes, représentent chacun l'hydrogène, un groupe alkyle en $C_1$-$C_6$, cycloalkyle en $C_5$-$C_6$, aryle en $C_6$-$C_{10}$ ou un groupe -$CH_2$-O-$R^5$, -$CH_2$-$CH_2$-$OR^5$ dans lesquels $R^5$ = hydrogène, alkyle en $C_1$-$C_6$, cycloalkyle en $C_5$-$C_6$, aryle en $C_6$-$C_{10}$ ou acyle en $C_1$-$C_6$ et

X est un nombre entier allant de 2 à 6,

en quantité de 0,1 à 15 % du poids du caoutchouc.

**2.** Procédé pour la préparation de vulcanisats de caoutchoucs chargés caractérisé en ce que l'on mélange

i) au moins un caoutchouc avec

ii) 10 à 120 de préférence 30 à 80 % en poids, par rapport au caoutchouc (i) d'une matière de charge et

iii) 0,1 à 15, de préférence 0,1 à 7,5 % en poids, par rapport au caoutchouc (i) de composés sulfurés de formule

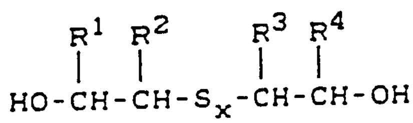

dans laquelle

$R^1$ à $R^4$, ayant des significations identiques ou différentes, représentent chacun l'hydrogène, un groupe alkyle en $C_1$-$C_6$, cycloalkyle en $C_5$-$C_6$, aryle en $C_6$-$C_{10}$ ou un groupe -$CH_2$-O-$R^5$, -$CH_2$-$CH_2$-O-$R^5$ dans lesquels $R^5$ = hydrogène, alkyle en $C_1$-$C_6$, cycloalkyle en $C_5$-$C_6$, aryle en $C_6$-$C_{10}$ ou acyle en $C_1$-$C_6$ et

X est un nombre entier allant de 2 à 6,

à des températures dans la masse d'au moins 120 °C et à des vitesses de cisaillement de 1 à 1000 $s^{-1}$, puis on vulcanise de la manière habituelle.

3. Oligosulfures de formules

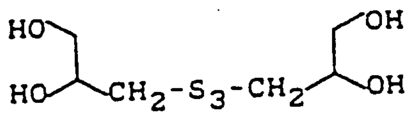

,

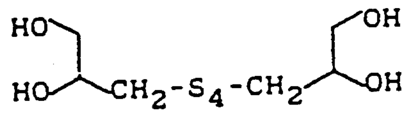

,

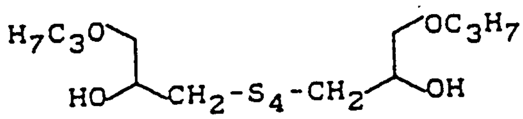

,

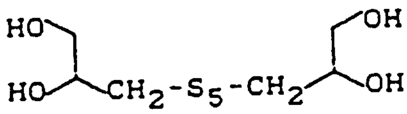

,

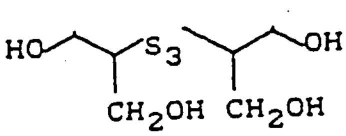

et

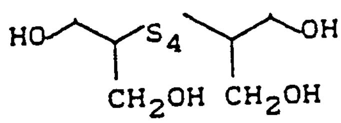

.

**Revendication pour l'Etat contractant suivant : ES**

1. Procédé pour la préparation de vulcanisats de caoutchoucs chargés caractérisé en ce que l'on mélange
   i) au moins un caoutchouc avec
   ii) 10 à 120, de préférence 30 à 80 % en poids, par rapport au caoutchouc (i) d'une matière de charge et
   iii) 0,1 à 15, de préférence 0,1 à 7,5 % en poids, par rapport au caoutchouc (i) de composés sulfurés de formule

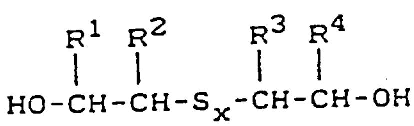

dans laquelle

$R^1$ à $R^4$, ayant des significations identiques ou différentes, représentent chacun l'hydrogène, un groupe alkyle en $C_1$-$C_6$, cycloalkyle en $C_5$-$C_6$, aryle en $C_6$-$C_{10}$ ou un groupe $-CH_2-O-R^5$, $-CH_2-CH_2-O-R^5$ dans lesquels $R^5$ = hydrogène, alkyle en $C_1$-$C_6$, cycloalkyle en $C_5$-$C_6$, aryle en $C_6$-$C_{10}$ ou acyle en $C_1$-$C_6$ et

X est un nombre entier allant de 2 à 6,

à des températures dans la masse d'au moins 120 °C et à des vitesses de cisaillement de 1 à 1000 $s^{-1}$, puis on vulcanise de la manière habituelle.